# DEMANDE DE BREVET EUROPEEN

(11) **EP 0 693 272 A1**
(43) Date de publication de la demande: **24.01.1996**
(21) Numéro de dépôt: 95420200.8
(22) Date de dépôt: 17.07.1995
(51) Int. Cl.: A61B 17/064, A61B 17/72

(54) **Dispositif de fixation pour ostéosynthèse**

(30) Priorité: 18.07.1994 FR 9409061
(71) Demandeur: ADVANCED TECHNICAL FABRICATION (Société Anonyme), F-74970 Marignier (FR); Noyer, Daniel, F-38300 Maubec (FR); ROUSSOULY, Pierre, F-69005 Lyon (FR)
(72) Inventeur: Godefroy, Jean, F-74130 Ayze (FR); Noyer, Daniel, F-38300 Maubec (FR); Roussouly, Pierre, F-69005 Lyon (FR)
(74) Mandataire: Poncet, Jean-François

(57) **Abrégé**

Le dispositif selon l'invention comprend au moins une portion axiale (1,21) de tige conformée pour pénétrer dans l'os (31) et se loger axialement dans le canal médullaire ou un canal osseux préformé, et au moins une portion latérale (5,25) de tige généralement parallèle à la portion axiale (1,21) de tige et conformée pour longer la surface extérieure de l'os. Un coude de liaison (3,23) relie les premières extrémités respectives des portions axiales (1,21) et latérales (5,25) de tige. Les secondes extrémités des portions latérales (5,25) de tige sont fixées à l'os par des vis osseuses (9,29) traversant des passages correspondants (28,128). Les portions axiales (1,21) de tige s'opposent aux efforts de cisaillement dans une zone intermédiaire d'ostéotomie ou de fracture (33), tandis que les portions latérales (5,25) de tige associées aux vis osseuses (9,29) et aux coudes de liaison (3,23) s'opposent aux efforts de traction dans la zone d'ostéotomie ou de fracture (33) de l'os. On assure ainsi une fixation correcte pour ostéosynthèse dans une zone d'ostéotomie ou de fracture.

## Description

La présente invention concerne les dispositifs de fixation qui sont utilisés pour l'ostéosynthèse d'une zone d'ostéotomie ou de fracture d'un os.

La consolidation osseuse d'une zone d'ostéotomie ou de fracture d'un os nécessite d'empêcher à la fois les mouvements d'amplitude notable dans le sens du cisaillement au niveau de la surface d'ostéotomie ou de fracture, et les mouvements axiaux d'amplitude notable dans le sens de l'écartement axial au niveau de la surface d'ostéotomie ou de fracture.

On utilise généralement pour cela deux types de moyens connus de fixation osseuse, à savoir les moyens de fixation rigides, et les moyens de fixation souples ou ostéosynthèse élastique.

Pour une fixation rigide, on utilise généralement des plaques vissées. La zone d'ostéotomie ou de fracture est alors immobilisée de façon rigide, empêchant le cisaillement et l'écartement dans la zone à maintenir. Il en résulte toutefois un excès de rigidité, qui s'oppose aux pressions axiales variables favorables à la consolidation osseuse, ce qui prolonge les durées de consolidation osseuse. Il faut en outre prévoir l'ablation du matériel d'ostéosynthèse après une durée de quelques mois, pour éviter que le caractère rigide du matériel modifie de façon durable la physiologie osseuse.

Pour une ostéosynthèse élastique, on utilise généralement soit des fils de cerclage, soit des broches, soit des agrafes comportant deux tiges latérales sensiblement parallèles reliées entre elles par une partie centrale formant pont, soit une combinaison de ces moyens. Les tiges latérales sont plantées dans l'os de part et d'autre de la zone d'ostéotomie ou de fracture. Un tel matériel n'interdit toutefois pas de façon satisfaisante les mouvements de cisaillement, et il est alors nécessaire de prévoir des moyens complémentaires d'immobilisation, par exemple un plâtre.

On connaît également des plaques pour fixation trochantérienne, qui viennent s'ancrer d'un côté de la zone d'ostéotomie ou de fracture sur la face supérieure du grand trochanter, qui longent l'os et sont fixées de l'autre côté de la zone d'ostéotomie ou de fracture sur la partie diaphysaire de l'os par une ou plusieurs vis. De telles plaques assurent un maintien correct en traction axiale sur l'os, mais ne permettent pas de reprendre les efforts de cisaillement. En outre, ces plaques ne conviennent qu'au trochanter, et peuvent gêner la vision radiographique de la fracture et/ou de la prothèse.

Le document WO-A-86 04798 NGUYEN décrit un matériel de tenue de fractures du col du fémur, ou fractures pertrochantériennes, composé d'une lame en gouttière à fente ovale intermédiaire, d'un clou flexible recourbé traversant la fente ovale de la lame, et d'une vis mécanique traversant un oeillet de l'extrémité du clou et un trou axial de la lame pour bloquer le clou dans la fente ovale de lame. Ce matériel est complexe, et n'est pas adapté pour tenir les fractures épiphysaires et métaphysaires des os longs.

Le document US-A-4 733 654 MARINO décrit un autre matériel, encore plus complexe et onéreux, pour tenir de façon rigide les fractures du col du fémur, avec une combinaison d'une plaque latérale, de plusieurs broches et de plusieurs vis mécaniques.

Le problème proposé par la présente invention est de définir un nouveau matériel qui soit à la fois simple, robuste, élastique, facile à réaliser, facile à mettre en place, et qui permette à la fois un maintien axial correct des deux parties d'os séparées par la zone d'ostéotomie ou de fracture, et un maintien correct en cisaillement au niveau de la zone d'ostéotomie ou de fracture.

Le matériel selon l'invention est principalement destiné aux fractures épiphysaires et métaphysaires des os longs tels que le tibia, l'humérus, le fémur, le cubitus, le radius. Le matériel convient également à la réduction des fractures des os courts tels que tarse, métatarse, phalange.

Pour atteindre ces objets ainsi que d'autres, le dispositif de fixation pour ostéosynthèse d'une zone d'ostéotomie ou de fracture d'un os selon l'invention comprend un élément principal en forme de tige recourbée en plusieurs portions constituant :
- au moins une portion axiale allongée de tige, conformée pour pénétrer dans l'os et pour être logée axialement dans un tunnel préformé intraosseux et/ou dans le canal médullaire de l'os,
- au moins une portion latérale allongée de tige, généralement parallèle à la portion axiale de tige, et conformée pour longer la surface extérieure de l'os,
- au moins un coude de liaison, reliant une première extrémité de la portion axiale de tige à une première extrémité correspondante de la portion latérale de tige, en les maintenant selon un écartement approprié; selon l'invention :
- le dispositif comprend au moins une vis osseuse à tête,
- au moins un passage transversal de vis osseuse est ménagé dans la portion latérale de tige au voisinage de sa seconde extrémité, et conformé pour sa fixation à l'os par ladite vis osseuse pénétrant transversalement dans l'os,
- les portions axiale et latérale de tige ont des longueurs suffisantes pour se développer de part et d'autre de la zone d'ostéotomie ou de fracture de l'os, la portion axiale de tige pouvant pénétrer dans l'os selon un premier côté de la zone d'ostéotomie ou de fracture de l'os tandis que la vis osseuse est logée dans l'os selon le second côté de la zone d'ostéotomie ou de fracture de l'os,

de sorte que la portion axiale de tige s'oppose aux efforts de cisaillement dans la zone d'ostéotomie ou de fracture de l'os, et la portion latérale de tige associée à la vis osseuse et au coude de liaison s'oppose aux efforts de traction dans la zone d'ostéotomie ou de fracture de l'os.

Selon un premier mode de réalisation, le dispositif selon l'invention comprend une portion axiale unique de tige, reliée par un coude de liaison à une portion latérale unique de tige dont la seconde extrémité est recourbée pour former un oeillet de passage de tige de vis osseuse.

Selon un second mode de réalisation, le dispositif de fixation selon l'invention comprend deux portions axiales de tige généralement parallèles l'une à l'autre, se raccordant par deux coudes de liaison parallèles à deux portions latérales de tige généralement parallèles dont les secondes extrémités sont reliées par une demi-boucle permettant le logement et le passage de la tige d'une vis osseuse.

Selon une variante, les secondes extrémités des deux portions latérales de tige sont reliées par deux demi-boucles permettant le logement et le passage de deux tiges de vis osseuses.

D'autres objets, caractéristiques et avantages de la présente invention ressortiront de la description suivante de modes de réalisation particuliers, faite en relation avec les figures jointes, parmi lesquelles:
- la figure 1 est une vue en perspective d'un dispositif de fixation pour ostéosynthèse selon un premier mode de réalisation de l'invention, avec une seule portion axiale de tige et une seule portion latérale de tige ;
- la figure 2 est une vue en perspective montrant un dispositif de fixation pour ostéosynthèse selon un second mode de réalisation de I'invention ;
- la figure 3 est une vue en perspective illustrant un dispositif de fixation pour ostéosynthèse selon un troisième mode de réalisation de l'invention ;
- la figure 4 illustre en perspective l'adaptation d'un dispositif de fixation selon l'invention pour le maintien du grand trochanter après pose de la partie fémorale d'une prothèse de hanche ;
- la figure 5 illustre en perspective l'adaptation d'un dispositif de fixation selon l'invention appliqué au maintien d'une fracture épiphysaire du cubitus ;
- les figures 6 et 7 illustrent en perspective deux autres modes de réalisation du dispositif de l'invention ;
- les figures 8 et 9 illustrent un dispositif selon l'invention avec structure d'appui selon un premier mode de réalisation ; et
- la figure 10 illustre un autre mode de réalisation de structure d'appui.

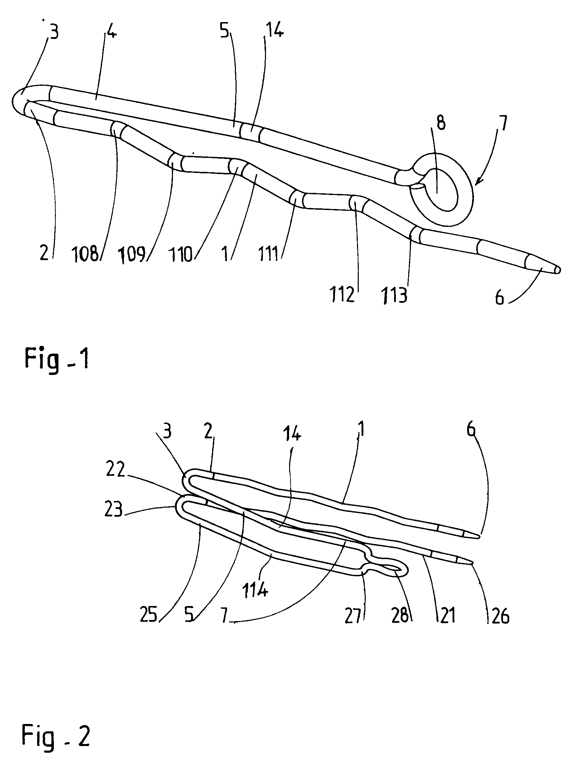

Dans le mode de réalisation illustré sur la figure 1, le dispositif de fixation pour ostéosynthèse selon l'invention comprend un élément principal constitué d'une tige recourbée pour former plusieurs portions comportant notamment une portion axiale 1 allongée de tige, de section réduite par rapport à sa longueur, de façon à être conformée pour être logée axialement dans le canal médullaire d'un os et/ou un tunnel préformé. La portion axiale 1 de tige se raccorde selon sa première extrémité 2, par un coude de liaison 3, à la première extrémité 4 d'une portion latérale 5 allongée de tige généralement parallèle à la portion axiale 1 de tige, et également de section réduite par rapport à sa longueur. Le coude de liaison 3 maintient les premières extrémités correspondantes 2 et 4 des portions 1 et 5 de tige selon un écartement approprié, correspondant sensiblement à l'épaisseur de l'os.

La seconde extrémité 6 de la portion axiale 1 de tige est de section réduite, pour faciliter sa pénétration dans l'os.

La seconde extrémité 7 de la portion latérale 5 de tige est recourbée pour former un oeillet 8 de passage pour la tige d'une vis osseuse ou pour un autre moyen de fixation, par exemple un fil de cerclage.

Dans le mode de réalisation illustré sur la figure, la portion axiale 1 de tige comporte des ondulations formées par une suite d'incurvations ou de pliages 108-113. Ces ondulations permettent d'optimiser les contacts de la portion axiale 1 de tige dans le canal médullaire d'un os, et assurent un freinage antirecul si le système de fixation n'est pas placé.

La portion latérale 5 de tige comprend au moins une courbure ou angulation intermédiaire 14, dont l'ouverture est orientée vers la portion axiale correspondante 1 de tige, pour présenter une certaine élasticité axiale dans la portion latérale 5 de tige entre l'oeillet 8 et le coude de liaison 3. Cela permet d'introduire une élasticité dans le maintien axial de la zone d'ostéotomie ou de fracture d'un os.

Selon un autre mode de réalisation, on peut également prévoir plusieurs ondulations ménagées dans la portion latérale 5 de tige, augmentant l'élasticité de maintien axial de la zone d'ostéotomie ou de fracture de l'os.

La portion axiale 1 de tige est avantageusement plus longue que la portion latérale 5 de tige.

Dans le mode de réalisation de la figure 2, le dispositif selon l'invention comprend deux portions axiales 1 et 21 de tige, généralement parallèles l'une à l'autre, se raccordant selon leurs premières extrémités 2 et 22 par deux coudes de liaison parallèles respectifs 3 et 23 à deux portions latérales de tige 5 et 25 généralement parallèles dont les secondes extrémités 7 et 27 sont reliées par une demi-boucle 28 permettant le logement et le passage de la tige d'une vis osseuse avec un jeu suffisant pour conférer une liberté d'orientation de la vis osseuse. Les portions axiales 1 et 21 de tige présentent également des ondulations, et les portions latérales 5 et 25 de tige présentent des courbures ou angulations intermédiaires respectives 14 et 114, comme dans le mode de réalisation de la figure 1. Les portions axiales 1 et 21 de tige sont plus longues que les portions latérales 5 et 25 de tige.

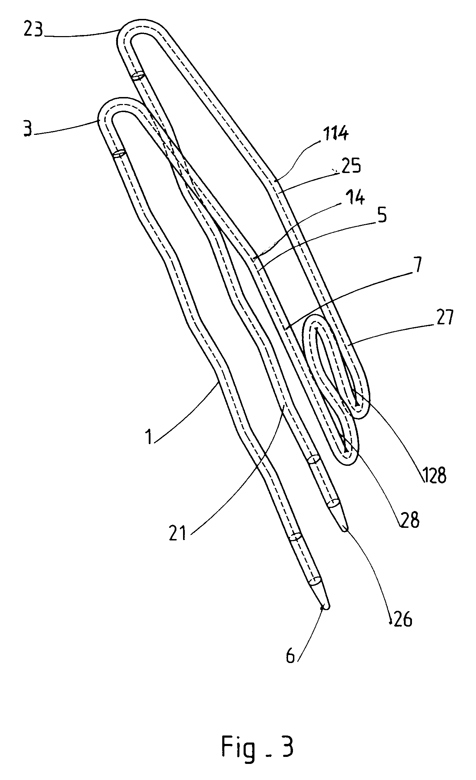

Dans le mode de réalisation de la figure 3, le dispositif selon l'invention comprend également deux portions axiales 1 et 21 de tige se raccordant par deux coudes de liaison respectifs 3 et 23 à deux portions latérales 5 et 25 de tige. Les secondes extrémités 7 et 27 des portions latérales 5 et 25 de tige sont reliées par deux demi-boucles 28 et 128 permettant le logement et le passage des tiges de deux vis osseuses ou d'autres moyens de fixation. Les portions axiales 1 et 21 de tige présentent des ondulations, et les portions latérales 5 et 25 de tige présentent chacune au moins une courbure ou angulation intermédiaire 14 ou 114, comme dans le mode de réalisation de la figure 1. Les portions axiales 1 et 21 de tige sont plus longues que les portions latérales 5 et 25 de tige.

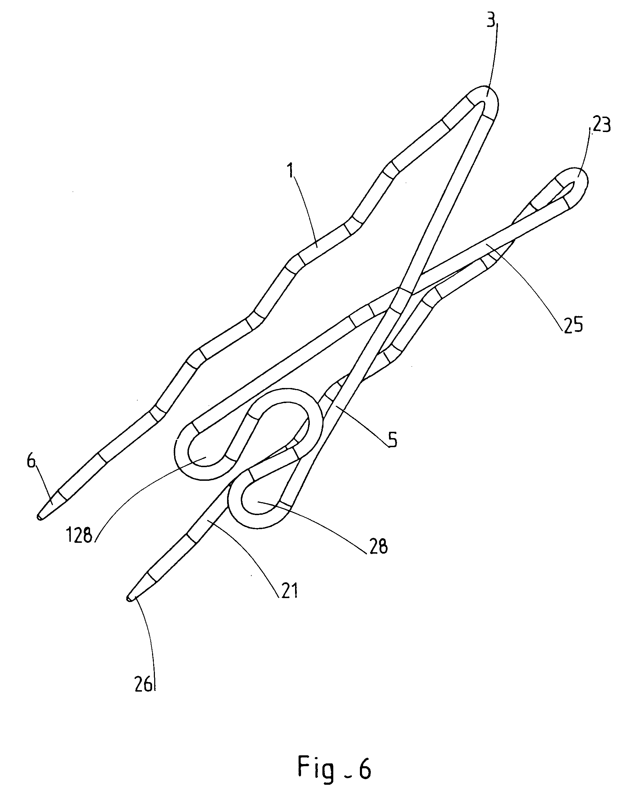

Dans la variante de la figure 6, le dispositif a sensiblement la même structure que dans le mode de réalisation de la figure 3, les caractéristiques correspondantes étant repérées par les mêmes références numériques sur le dessin, avec en outre un croisement des portions latérales 5 et 25 de tige dans leur zone intermédiaire.

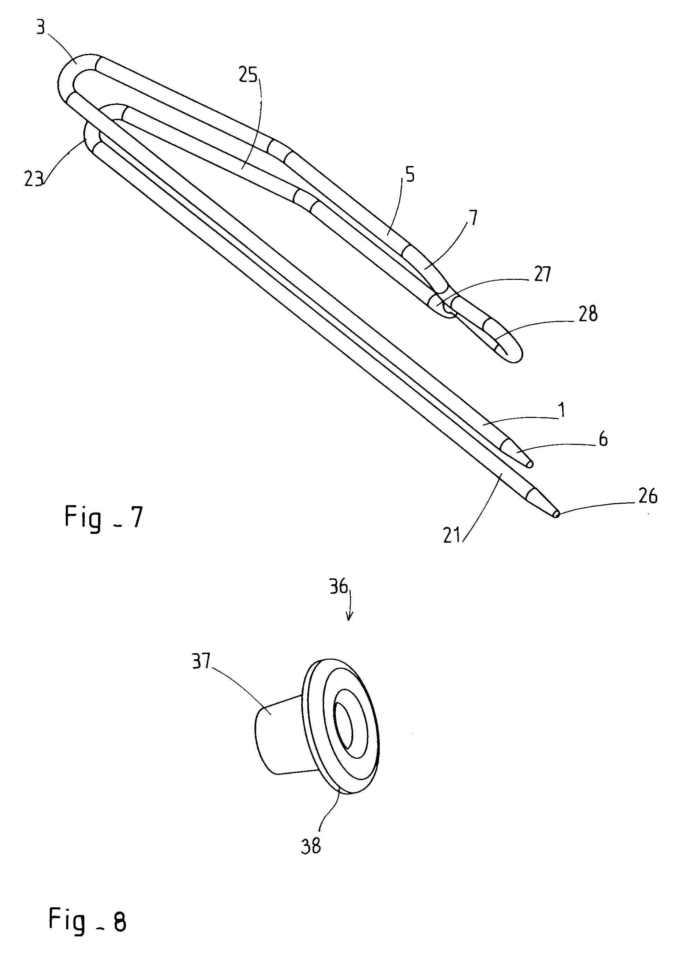

Le mode de réalisation de la figure 7 est similaire de celui de la figure 2, et les éléments correspondants sont repérés par les mêmes références numériques. La différence est que les portions axiales 1 et 21 de tige sont sensiblement rectilignes. On notera que de telles portions axiales rectilignes de tige peuvent être utilisées dans tous les autres modes de réalisation représentés sur les figures.

Dans tous les modes de réalisation représentés, l'élément principal du dispositif est constitué d'un fil en matériau approprié, de section sensiblement constante, dont les extrémités 6 ou 26 sont réduites.

Un dispositif de fixation selon l'invention est réalisé par un procédé comprenant une étape au cours de laquelle on forme un fil en matériau approprié selon une ou plusieurs portions constituant les portions axiales 1 ou 21 de tige, avec une ou plusieurs portions constituant les portions latérales 5 ou 25 de tige, avec les coudes de liaison 3 ou 23, et avec les oeillets 8 ou demi-boucles 28 et 128 de passage de vis.

Les portions axiales 1 et 21 et latérales 5 et 25 de tige ont une longueur suffisante pour se développer de part et d'autre de la zone d'ostéotomie ou de fracture d'un os à traiter. On pourra avantageusement utiliser un élément principal dont la ou les portions latérales 5 et 25 de tige ont une longueur au moins égale aux 2/3 de la longueur des portions axiales 1 et 21 de tige.

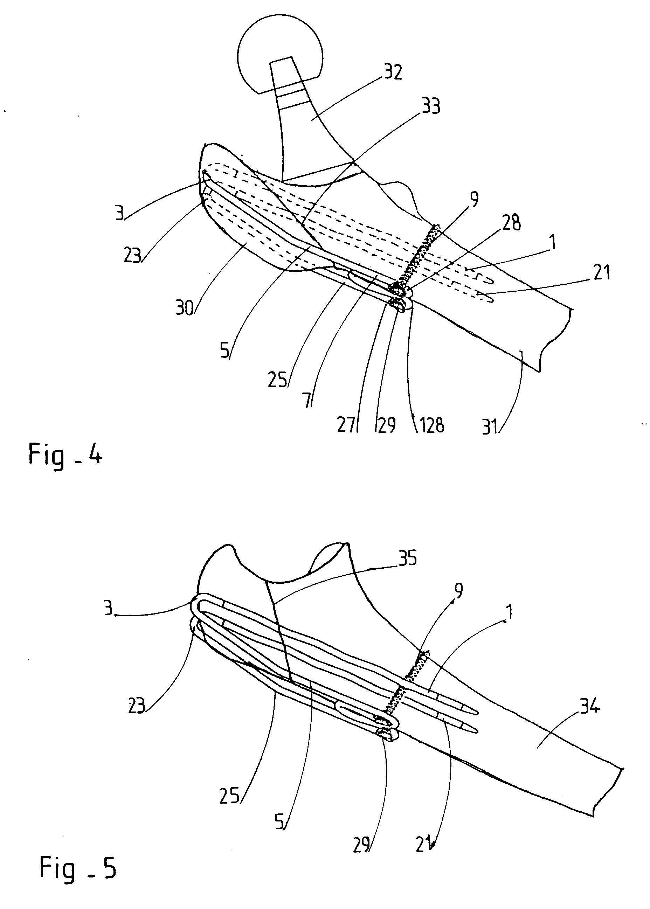

Les figures 4 et 5 illustrent deux utilisations d'un dispositif de fixation selon l'invention.

Sur la figure 4, il s'agit du maintien du grand trochanter 30 sur le fémur 31 après pose d'une partie fémorale de prothèse de hanche 32. On distingue les portions axiales 1 et 21 de tige, engagées axialement de part et d'autre de la prothèse dans le canal médullaire du fémur 31, dans lequel elles pénètrent depuis la partie supérieure du grand trochanter. On distingue le coude de liaison 3 qui pénètre dans le grand trochanter 30. Les portions latérales 5 et 25 de tige longent la surface externe du fémur 31, de part et d'autre de la zone d'ostéotomie 33, de façon que les coudes de liaison 3 et 23 se situent d'un côté de la zone d'ostéotomie 33, et les secondes extrémités 7 et 27 des portions latérales 5 et 25 de tige se situent du côté opposé de la zone d'ostéotomie 33. Deux vis osseuses 9 et 29 traversent les demi-boucles 28 et 128 respectives du dispositif, et sont vissées dans la partie diaphysaire du fémur 31, comme illustré sur la figure. On comprend que les portions axiales 1 et 21 de tige s'opposent aux efforts de cisaillement dans la zone d'ostéotomie 33 de l'os, tandis que les portions latérales 5 et 25 de tige associées aux coudes de liaison 3 et 23 et aux vis osseuses 9 et 29 s'opposent aux efforts de traction dans la zone d'ostéotomie 33 de l'os.

Sur la figure 5, on retrouve le même dispositif avec les portions axiales 1 et 21 de tige engagées dans le canal médullaire d'un cubitus 34 présentant une zone de fracture 35. Les coudes 3 et 23 pénètrent dans l'os selon un premier côté de la zone de fracture 35, tandis que les vis 9 et 29 sont fixées dans la partie diaphysaire du cubitus 34 à l'opposé de la zone de fracture 35.

Dans certaines applications, les coudes de liaison 3 et 23 exercent sur l'os une pression conséquente, pouvant détériorer localement l'os et modifier les conditions de serrage exercées par le dispositif sur la zone d'ostéotomie ou de fracture de l'os 33, 35.

Cette pression locale peut avantageusement être réduite en prévoyant, dans la zone des coudes de liaison 3 et 23, une structure d'appui présentant une surface élargie d'appui sur l'os.

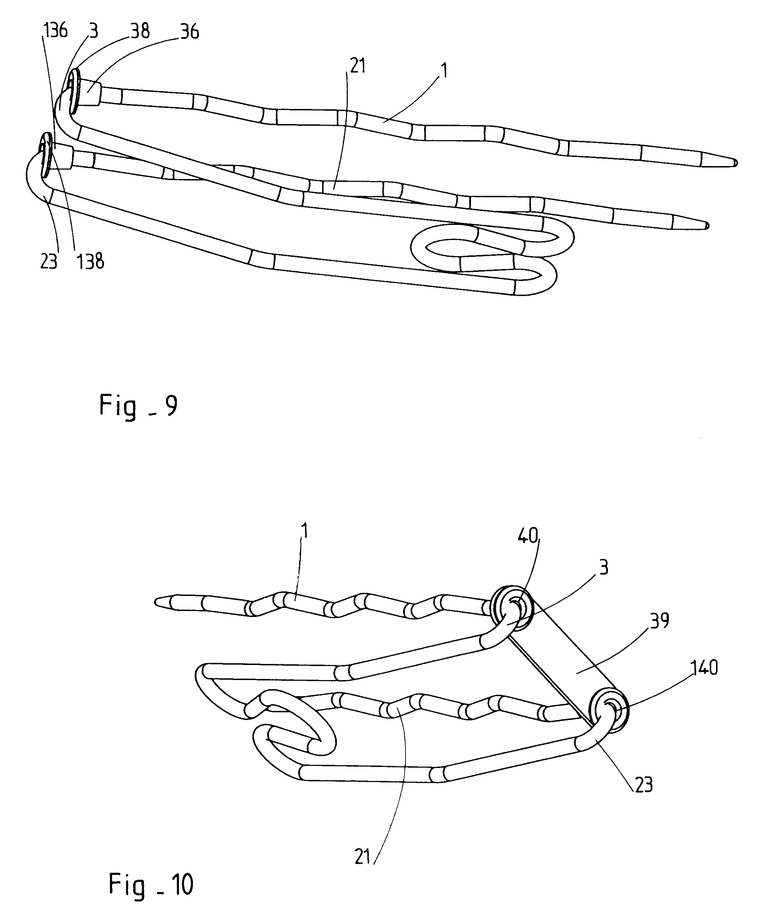

Un mode de réalisation d'une telle structure d'appui est illustré sur la figure 8, sous forme d'une douille épaulée 36 comportant un fût tronconique 37 de pénétration avec une partie d'appui 38 élargie. Comme illustré sur la figure 9, on adapte une telle douille épaulée 36 ou 136 sur chaque portion axiale 1 ou 21 de tige, orientée de façon que la partie d'appui 38 ou 138 vienne en appui contre le coude correspondant 3 ou 23.

Un autre mode de réalisation d'une telle structure d'appui est illustré sur la figure 10, sous forme d'une plaque d'appui 39 percée de deux trous 40 et 140. La plaque 39 est engagée sur les deux portions axiales 1 et 21 de tige jusqu'à porter contre les coudes 3 et 23.

La présente invention n'est pas limitée aux modes de réalisation qui ont été explicitement décrits, mais elle en inclut les diverses variantes et généralisations contenues dans le domaine des revendications ci-après.

## Revendications

**1 -** Dispositif de fixation pour ostéosynthèse d'une zone d'ostéotomie ou de fracture (33,35) d'un os, comprenant un élément principal en forme de tige recourbée en plusieurs portions constituant :
- au moins une portion axiale (1,21) allongée de tige, conformée pour pénétrer dans l'os (31,34) et pour être logée axialement dans un tunnel préformé intraosseux et/ou dans le canal médullaire de l'os,
- au moins une portion latérale (5,25) allongée de tige, généralement parallèle à la portion axiale (1,21) de tige, et conformée pour longer la surface extérieure de l'os,
- au moins un coude de liaison (3,23), reliant une première extrémité (2,22) de la portion axiale (1,21) de tige à une première extrémité correspondante de la portion latérale (5,25) de tige, en les maintenant selon un écartement approprié,
caractérisé en ce que :
- le dispositif comprend au moins une vis osseuse (9,29) à tête,
- au moins un passage transversal de vis osseuse (8,28,128) est ménagé dans la portion latérale (5,25) de tige au voisinage de sa seconde extrémité (7,27), et conformé pour sa fixation à l'os par ladite vis osseuse (9,29) pénétrant transversalement dans l'os,
- les portions axiale et latérale de tige (1,21,5,25) ont des longueurs suffisantes pour se développer de part et d'autre de la zone d'ostéotomie ou de fracture (33,35) de l'os, la portion axiale (1,21) de tige pouvant pénétrer dans l'os selon un premier côté de la zone d'ostéotomie ou de fracture de l'os (33,35) tandis que la vis osseuse (9,29) est logée dans l'os selon le second côté de la zone d'ostéotomie ou de fracture (33,35) de l'os,
de sorte que la portion axiale (1,21) de tige s'oppose aux efforts de cisaillement dans la zone d'ostéotomie ou de fracture (33,35) de l'os, et la portion latérale (5,25) de tige associée à la vis osseuse (9,29) et au coude de liaison (3,23) s'oppose aux efforts de traction dans la zone d'ostéotomie ou de fracture (33,35) de l'os.

**2 -** Dispositif selon la revendication 1, caractérisé en ce qu'il comprend une portion axiale unique (1) de tige reliée par un coude de liaison (3) à une portion latérale unique (5) de tige dont la seconde extrémité (7) est recourbée pour former un oeillet (8) de passage de tige de vis osseuse.

**3 -** Dispositif selon la revendication 1, caractérisé en ce qu'il comprend deux portions axiales (1,21) de tige généralement parallèles l'une à l'autre, se raccordant par deux coudes de liaison parallèles (3,23) à deux portions latérales (5,25) de tige généralement parallèles dont les secondes extrémités (7,27) sont reliées par une demi-boucle (28) permettant le logement et le passage de la tige d'une vis osseuse.

**4 -** Dispositif selon la revendication 1, caractérisé en ce qu'il comprend deux portions axiales (1,21) de tige généralement parallèles l'une à l'autre, se raccordant par deux coudes de liaison parallèles (3,23) à deux portions latérales (5,25) de tige généralement parallèles dont les secondes extrémités (7,27) sont reliées par deux demi-boucles (28,128) permettant le logement et le passage de deux tiges de vis osseuses (9,29).

**5 -** Dispositif selon l'une quelconque des revendications 1 à 4, caractérisé en ce que chaque portion axiale (1,21) de tige comporte des ondulations (108-113) permettant d'optimiser les contacts dans le canal médullaire ou tunnel préformé dans l'os et assurant un freinage antirecul.

**6 -** Dispositif selon l'une quelconque des revendications 1 à 5, caractérisé en ce que chaque portion latérale (5,25) de tige comprend des ondulations permettant d'introduire une élasticité dans le maintien axial de la zone d'ostéotomie ou de fracture (33,35).

**7 -** Dispositif selon l'une quelconque des revendications 1 à 6, caractérisé en ce que chaque portion latérale (5,25) de tige comprend une courbure ou angulation intermédiaire (14, 114) dont l'ouverture est orientée vers la portion axiale correspondante (1,21) de tige.

**8 -** Dispositif selon l'une quelconque des revendications 1 à 7, caractérisé en ce que les portions axiales (1,21) de tige sont plus longues que les portions latérales (5,25) de tige.

**9 -** Dispositif selon l'une quelconque des revendications 1 à 8, constitué d'un fil en matériau approprié de section sensiblement constante dont les extrémités (6,26) sont réduites.

**10 -** Dispositif selon l'une quelconque des revendications 1 à 9, caractérisé en ce qu'il comprend en outre, dans la zone des coudes de liaison (3, 23), une structure d'appui (36, 39) présentant une surface élargie d'appui sur l'os.

**11 -** Procédé de réalisation d'un dispositif de fixation pour ostéosynthèse selon l'une quelconque des revendications 1 à 10, caractérisé en ce qu'il comprend une étape au cours de laquelle on forme un fil en matériau approprié avec une ou plusieurs portions constituant les portions axiales (1,21) de tige, avec une ou plusieurs portions constituant les portions latérales (5,25) de tige, avec des coudes de liaison (3,23) et avec des oeillets (8) ou demi-boucles (28,128) de passage de vis.
